# EUROPEAN PATENT SPECIFICATION

(11) **EP 4 714 355 B1**
(45) Date of publication and mention of the grant of the patent: **26.08.2026**
(21) Application number: 25203764.3
(22) Date of filing: 22.09.2025
(51) Int. Cl.: A61B 5/287, A61B 5/367, A61B 5/00, A61B 18/14

(54) **CREATING ELECTRO-ANATOMICAL MAPPINGS OF CARDIAC TISSUE USING FAR FIELD SIGNALS RECEIVED AT MULTIPLE DISTANCES FROM AN ELECTRODE**
ERZEUGUNG ELEKTROANATOMISCHER ABBILDUNGEN VON HERZGEWEBE UNTER VERWENDUNG VON FERNFELDSIGNALEN, DIE IN MEHREREN ABSTÄNDEN VON EINER ELEKTRODE EMPFANGEN WERDEN
CRÉATION DE CARTOGRAPHIES ÉLECTRO-ANATOMIQUES DE TISSU CARDIAQUE À L'AIDE DE SIGNAUX DE CHAMP LOINTAIN REÇUS À DE MULTIPLES DISTANCES D'UNE ÉLECTRODE

(30) Priority: 23.09.2024 US 202463697933 P; 19.09.2025 US 202519334568
(43) Date of publication of application: 25.03.2026
(73) Proprietor: Biosense Webster (Israel) Ltd., 2066717 Yokneam (IL)
(72) Inventor: TSOREF, Liat, 2066717 Yokneam (IL); PFEFFER, Shmuel Yitzhak, 2066717 Yokneam (IL); RODRIGUEZ, Haim, 2066717 Yokneam (IL)
(74) Representative: Carpmaels & Ransford LLP

(56) References cited:
- US-A1- 2020 281 494
- US-A1- 2023 181 087
- BEHESHTI MOHAMMADALI ET AL: "Determinants of atrial bipolar voltage: Inter electrode distance and wavefront angle", COMPUTERS IN BIOLOGY AND MEDICINE, NEW YORK, NY, US, vol. 102, 27 July 2018 (2018-07-27), pages 449 - 457, XP085515312, ISSN: 0010-4825, DOI: 10.1016/J.COMPBIOMED.2018.07.011

## Description

### Related Applications

The present disclosure is directed to several improved techniques for analyzing cardiac signals, and claims priority to U.S. Provisional Patent Application No. 63/697,933, filed September 23, 2024, entitled "Methods For Analyzing Cardiac Signals And Generating Electro-Anatomical Maps Of The Heart,". The disclosed techniques also relate to build on concepts discussed in pending U.S. Patent Application No. 18/072,793, filed December 1, 2022, entitled "Intracardiac Unipolar Far Field Cancelation Using Multiple Electrode Catheters" (pending), U.S. Patent Application No. 18/756,903, filed June 27, 2024, entitled "Intracardiac Unipolar Far Field Cancelation Using Multiple Electrode Catheters And Methods For Creating An Ecg Depth And Radial Lens" (pending), U.S. Patent Application No. 19/238,791, filed June 16, 2025, entitled "System and Method for Far-field Voltage Mapping for Scar Severity Estimation" and U.S. Patent Application No. 18/505,956 entitled "Catheter With Flexible Polymer As Outer Support Structure," filed November 9, 2023 (pending). These existing applications provide relevant background to the techniques disclosed herein. The improvements disclosed herein may be used in conjunction with techniques disclosed in the above applications.

### Technical Field

This disclosure is related to generating, visualizing, and displaying information representative of cardiac signals. More particularly, systems and methods are disclosed for generating, visualizing, and displaying cardiac signal information present at different depths of cardiac tissue.

### Background

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., pulsed field or radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The electrodes may be placed in contact with cardiac tissue or other vascular tissue and then activated with RF, pulsed field, or other energy to thereby ablate the contacted tissue. In some cases, the electrodes may be bipolar. In some other cases, a monopolar electrode may be used in conjunction with a ground pad or other reference electrode that is in contact with the patient.

Examples of ablation catheters are described in U.S. Pub. No. 2013/0030426, entitled "Integrated Ablation System using Catheter with Multiple Irrigation Lumens," published Jan. 31, 2013; U.S. Pub. No. 2017/0312022, entitled "Irrigated Balloon Catheter with Flexible Circuit Electrode Assembly," published Nov. 2, 2017; U.S. Pub. No. 2018/0071017, entitled "Ablation Catheter with a Flexible Printed Circuit Board," published Mar. 15, 2018; U.S. Pub. No. 2018/0056038, entitled "Catheter with Bipole Electrode Spacer and Related Methods," published Mar. 1, 2018; U.S. Pat. No. 10,130,422, entitled "Catheter with Soft Distal Tip for Mapping and Ablating Tubular Region," issued Nov. 20, 2018; U.S. Pat. No. 8,956,353, entitled "Electrode Irrigation Using Micro-Jets," issued Feb. 17, 2015; and U.S. Pat. No. 9,801,585, entitled "Electrocardiogram Noise Reduction," issued Oct. 31, 2017.

Some catheter ablation procedures may be performed after using electrophysiology (EP) mapping to identify tissue regions that should be targeted for ablation. Such EP mapping may include the use of sensing microelectrodes on a catheter (e.g., the same catheter that is used to perform the ablation or a dedicated mapping catheter). Such sensing microelectrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued Apr. 14, 1998. Examples of EP mapping catheters are described in U.S. Pat. No. 9,907,480, entitled "Catheter Spine Assembly with Closely-Spaced Bipole Microelectrodes," issued Mar. 6, 2018; U.S. Pat. No. 10,130,422, entitled "Catheter with Soft Distal Tip for Mapping and Ablating Tubular Region," issued Nov. 20,2018; and U.S. Pub. No. 2018/0056038, entitled "Catheter with Bipole Electrode Spacer and Related Methods," published Mar. 1, 2018.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued Nov. 1, 2016; and various other references that are cited herein.

While various catheter systems and methods have been described, none are believed to provide the features described herein.

.

### Summary of the Disclosure

This disclosure is directed toward the creation of multiple signal mappings of cardiac tissue based on signals derived from different sets of electrodes. In one example, electrical activity is received from a plurality of electrodes of a multi-electrode catheter within a heart of a patient. For a first location within the heart: (i) a first electrode is selected from the plurality of electrodes; (ii) a first set of electrodes is selected, each of which is within a first distance of the first electrode, wherein the first set of electrodes does not include the first electrode; (iii) a first spatial electrode signal analysis is performed of the electrical activity of the first electrode based on signals from the first set of electrodes; (iv) a second set of electrodes is selected each of which is within a second distance of the first electrode, the second distance being greater than the first distance, wherein the second set of electrodes does not include the first electrode; and (v) a second spatial electrode signal analysis is performed of the electrical activity of the first electrode in accordance with signals from the second set of electrodes. This process is repeated for a plurality of further locations within the heart, and used to generate at least first and second electro-anatomical maps of the heart. The maps depict electrical activity of the cardiac tissue corresponding to the first location and the plurality of further locations, wherein the first electro-anatomical map depicts electrical activity identified based on the first spatial electrode analysis and the second electro-anatomical map depicts electrical activity identified based on the second spatial electrode signal analysis. Alternatively, the first electro-anatomical map depicts scar tissue identified based on the first spatial electrode analysis and the second electro-anatomical map depicts scar tissue identified based on the second spatial electrode signal analysis.

In some examples, the first location and the plurality of further locations are at a common depth of cardiac tissue within the heart, and the first and second electroanatomical maps each depict a different analysis of electrical signals within the common depth. In some examples, the first spatial electrode signal analysis and the second spatial signal analysis are performed using a suitable artificial intelligence model including, for example, a previously trained neural network.

In some examples, the first spatial electrode signal analysis is based at least in part on the first distance, and the second spatial electrode signal analysis is based at least part on the second distance. In some such examples, far field signals are identified based on the first spatial analysis and the second spatial analysis, and the far field signals are subtracted from electrical signals from the first electrode. In some examples, the far field signals are determined from the signals from the first set of electrodes based on the first distance and from the signals from the second set of electrodes based on second distance.

In some examples, the first electrode is at the first location within the heart, signals emanate from the first location along a first direction, and the first and second sets of electrodes are aligned at an angle perpendicular to the first direction. In some examples, the first set of electrodes and the second set of electrodes are arranged on separate planes and separated by a dielectric layer. In some examples, the multi-electrode catheter comprises at least one pair of electrodes which are spaced apart from each other by a distance which is smaller than dimensions of the electrodes in the pair.

Examples herein improve existing computer-based electroanatomical mapping systems. In particular, by decomposing signals into near-field and far-field components from selected electrodes at varying distances in real time, the disclosed system improves the functioning of the computer itself enabling more accurate and insightful maps than could be generated using conventional systems or manual human analysis. Such improvements are rooted in technology and address problems unique to electrophysiological signal processing, including cancellation of far-field interference and enhancement of local activation detection.

### Brief Description Of The Drawings

A more detailed understanding may be had from the following description, given by way of example in conjunction with the accompanying drawings, wherein like reference numerals in the figures indicate like elements, and wherein:
FIG. 1 shows an example catheter-based electrophysiology mapping and ablation system according to one or more embodiments;
FIG. 2 is a block diagram of an example system for remotely monitoring and communicating patient biometrics according to one or more embodiments;
FIG. 3 is a system diagram of an example of a computing environment in communication with a network according to one or more embodiments;
FIG. 4 shows an example of a multi-electrode catheter design for implementing techniques disclosed herein according to one or more embodiments;
FIG. 5 shows a further example of a multi-electrode catheter design for implementing techniques disclosed herein according to one or more embodiments;
FIG. 5A shows a further example of a multi-electrode catheter design for implementing techniques disclosed herein wherein the unipolar electrodes on the catheter of FIG. 5 are replaced with bi-polar split electrodes;
FIG. 6A shows a further example of a multi-electrode catheter design for implementing techniques disclosed herein according to one or more embodiments;
FIG. 6B shows a further example of a multi-electrode catheter design for implementing techniques disclosed herein according to one or more embodiments;
FIG. 7 is a multi-electrode catheter depicting bi-polar split electrodes arranged within or along circles surrounding a selected electrode and used for implementing techniques disclosed herein according to one or more embodiments;
FIG. 8 is a multi-electrode catheter depicting bi-polar split electrodes aligned at, or with respect to, an angle perpendicular to the direction of signal propagation from a selected electrode according to one or more embodiments;

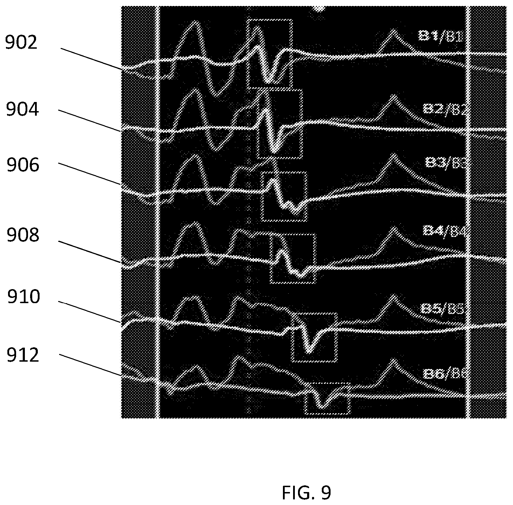

FIG. 9 is a signal diagram illustrating an exemplary application of the disclosed techniques for unipolar electrodes on a multi-electrode catheter;
FIG. 10A depicts an electro-anatomical map generated using the disclosed techniques according to one or more embodiments;
FIG. 10B depicts a further electro-anatomical map generated using the disclosed techniques according to one or more embodiments;
FIG. 11 illustrates an exemplary method for training and applying a machine learning algorithm to decompose near and far field components from measured signals according to one or more embodiments; and
FIG. 12 is a flow diagram illustrating a method for generating electroanatomical maps of the heart depicting electrical activity of the cardiac tissue or scar tissue based on the spatial electrode analysis techniques disclosed herein according to one or more embodiments.

### Detailed Description

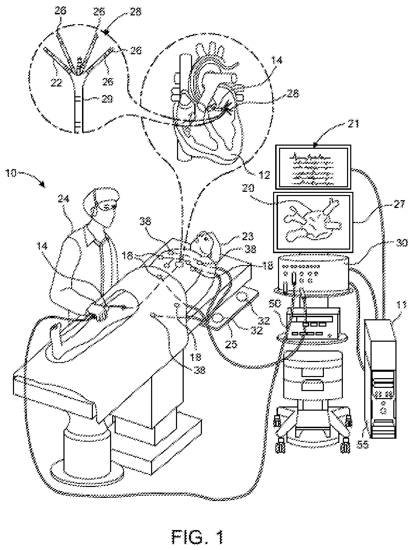

The techniques disclosed below are applied at different locations within the heart as a catheter moves through a patient's heart during a procedure in order to generate improved electro-anatomical maps of the heart. In one example, first and second electro-anatomical maps of the heart are generated, where the first electroanatomical map depicts electrical activity identified based on a first spatial electrode analysis and the second electro-anatomical map depicts electrical activity identified based on a second spatial electrode signal analysis. Alternatively, the first electroanatomical map depicts scar tissue (e.g., areas of low conductivity) identified based on the first spatial electrode analysis and the second electro-anatomical map depicts scar tissue (e.g., areas of low conductivity) identified based on the second spatial electrode signal analysis. Reference is made to FIG. 1 showing an example system (e.g., medical device equipment and/or catheter-based electrophysiology mapping and ablation), shown as system 10, in which one or more features of the subject matter herein can be implemented according to one or more embodiments. The system 10, as illustrated, includes a recorder 11, a heart 12, a catheter 14, a model or anatomical map 20, an electrogram 21, a spline 22, a patient 23, a physician 24 (or a medical professional, healthcare provider, or clinician), a location pad 25, an electrode 26, a display device 27, a distal tip 28, a sensor 29, a coil 32, a patient interface unit (PIU) 30, an electrode skin patches 38, an ablation energy generator 50, and a workstation 55. Note further each element and/or item of the system 10 is representative of one or more of that element and/or that item. The example of the system 10 shown in FIG. 1 can be modified to implement the embodiments disclosed herein. The disclosed embodiments can similarly be applied using other system components and settings. Additionally, the system 10 can include additional components, such as elements for sensing electrical activity, wired or wireless connectors, processing and display devices, or the like.

The system 10 includes multiple catheters 14, which are percutaneously inserted by the physician 24 through the patient's vascular system into a chamber or vascular structure of the heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters 14 may include catheters dedicated for sensing Intracardiac Electrogram (IEGM) signals, catheters dedicated for ablating and/or catheters dedicated for both sensing and ablating. The example catheter 14 that is configured for sensing IEGM is illustrated herein. The physician 24 brings the distal tip 28 of the catheter 14 into contact with the heart wall for sensing a target site in the heart 12. For ablation, the physician 24 would similarly bring a distal end of an ablation catheter to a target site for ablating.

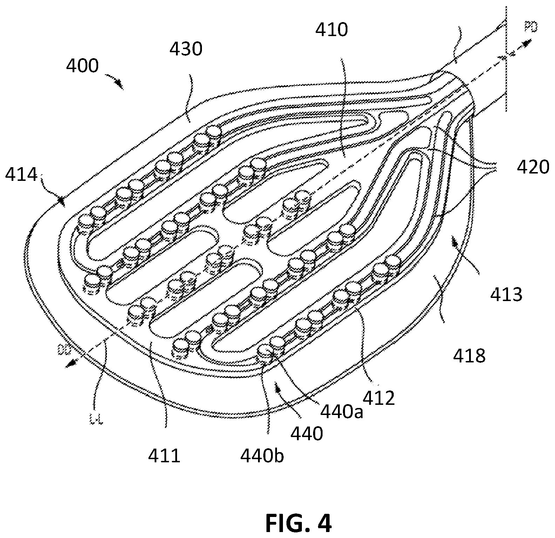

In FIG. 1, catheter 14 is depicted as including multiple electrodes 26 optionally distributed over a plurality of splines 22 at the distal tip 28 and configured to sense the IEGM signals. In some examples, catheter 14 is a multi-electrode configured to sense cardiac signals. Exemplary multi-electrode catheters for implementing the mapping techniques disclosed herein are shown in FIGs. 4-7 and discussed in further detail below. The catheter 14 may additionally include the sensor 29 embedded in or near the distal tip 28 for tracking position and orientation of the distal tip 28. Optionally and preferably, position sensor 29 is a magnetic based position sensor including three magnetic coils for sensing three-dimensional (3D) position and orientation. The catheter 14 may be a Pulsed-Field Ablation (PFA) catheter..

The sensor 29 (e.g., a position or a magnetic based position sensor) may be operated together with the location pad 25 including a plurality of magnetic coils 32 configured to generate magnetic fields in a predefined working volume. Real time position of the distal tip 28 of the catheter 14 may be tracked based on magnetic fields generated with the location pad 25 and sensed by the sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38 positioned for skin contact on the patient 23 to establish location reference for the location pad 25 as well as impedance-based tracking of the electrodes 26. For impedance-based tracking, electrical current is directed toward the electrodes 26 and sensed at the patches 38 (e.g., electrode skin patches) so that the location of each electrode can be triangulated via the patches 38. Details of the impedance-based location tracking technology are described in US Patent Nos. 7,536,218; 7,756,576; 7,848,787; 7,869,865; and 8,456,182.

The recorder 11 displays the electrograms 21 captured with the electrodes 18 (e.g., body surface electrocardiogram (ECG) electrodes) and intracardiac electrograms (IEGM) captured with the electrodes 26 of the catheter 14. The recorder 11 may include pacing capability for pacing the heart rhythm and/or may be electrically connected to a standalone pacer.

The system 10 may include the ablation energy generator 50 that is adapted to conduct ablative energy to the one or more of electrodes 26 at the distal tip 28 of the catheter 14 configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The PIU 30 is an interface configured to establish electrical communication between catheters, electrophysiological equipment, power supply and the workstation 55 for controlling operation of the system 10. Electrophysiological equipment of the system 10 may include for example, multiple catheters 14, the location pad 25, the body surface ECG electrodes 18, the electrode patches 38, the ablation energy generator 50, and the recorder 11. Optionally and preferably, the PIU 30 additionally includes processing capability for implementing real-time computations of location of the catheters and for performing ECG calculations.

The workstation 55 includes memory, processor unit with memory or storage with appropriate operating software loaded therein, and user interface capability. The workstation 55 may provide multiple functions, optionally including (1) modeling the endocardial anatomy in three-dimensions (3D) and rendering the model or anatomical map 20 for display on the display device 27, (2) displaying on the display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on the display device 27 sites of interest such as places where ablation energy has been applied. One commercial product embodying elements of the system 10 is available as the CARTOTM 3 System, available from Biosense Webster, Inc., 31A Technology Drive, Irvine, CA 92618.

For instance, the system 10 can be part of a surgical system (e.g., CARTO^{®} system sold by Biosense Webster) that is configured to obtain biometric data (e.g., anatomical and electrical measurements of a patient's organ, such as the heart 12 and as described herein) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 12. Such locating may be done via an electrophysiological investigation during which electrical potentials are detected spatially resolved with a mapping catheter (e.g., the catheter 14) introduced into the chamber of the heart 12. This electrophysiological investigation, the so-called electro-anatomical mapping, thus provides 3D mapping data which can be displayed on the display device 27. In many cases, the mapping function and a treatment function (e.g., ablation) are provided by a single catheter or group of catheters such that the mapping catheter also operates as a treatment (e.g., ablation) catheter at the same time.

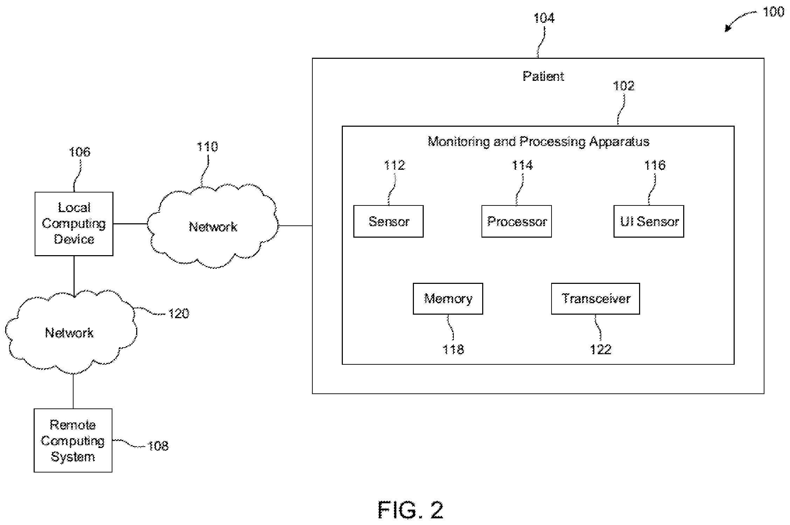

FIG. 2 is a block diagram of an example system 100 for remotely monitoring and communicating patient biometrics (i.e., patient data). In the example illustrated in FIG. 2, the system 100 includes a patient biometric monitoring and processing apparatus 102 associated with a patient 104, a local computing device 106, a remote computing system 108, a first network 110, a patient biometric sensor 112, a processor 114, a user input (UI) sensor 116, a memory 118, a second network 120, and a transmitter-receiver (i.e., transceiver) 122.

According to an embodiment, the patient biometric monitoring and processing apparatus 102 may be an apparatus that is internal to the patient's body (e.g., subcutaneously implantable), such as the catheter 14 of FIG. 1. The patient biometric monitoring and processing apparatus 102 may be inserted into a patient via any applicable manner including orally injecting, surgical insertion via a vein or artery, an endoscopic procedure, or a laparoscopic procedure.

According to an embodiment, the patient biometric monitoring and processing apparatus 102 may be an apparatus that is external to the patient, such as the electrode patches 38 of FIG. 1. For example, as described in more detail below, the patient biometric monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, the patient biometric monitoring and processing apparatus 102 may include both components that are internal to the patient and components that are external to the patient.

The single patient biometric monitoring and processing apparatus 102 is shown in FIG. 2. Example systems may, however, may include a plurality of patient biometric monitoring and processing apparatuses. A patient biometric monitoring and processing apparatus may be in communication with one or more other patient biometric monitoring and processing apparatuses. Additionally or alternatively, a patient biometric monitoring and processing apparatus may be in communication with the network 110.

One or more patient biometric monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other patient biometric monitoring and processing apparatuses 102. The additional information may be, for example, diagnosis information and/or additional information obtained from an additional device such as a wearable device. Each of the patient biometric monitoring and processing apparatus 102 may process data, including its own acquired patient biometrics as well as data received from one or more other patient biometric monitoring and processing apparatuses 102.

Biometric data (e.g., patient biometrics, patient data, or patient biometric data) can include one or more of local activation times (LATs), electrical activity, topology, bipolar mapping, reference activity, ventricle activity, dominant frequency, impedance, or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, intracardiac electrocardiogram (IC ECG) data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treat any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

In FIG. 2, the network 110 is an example of a short-range network (e.g., local area network (LAN), or personal area network (PAN)). Information may be sent, via the network 110, between the patient biometric monitoring and processing apparatus 102 and the local computing device 106 using any one of various short-range wireless communication protocols, such as Bluetooth, Wi-Fi, Zigbee, Z-Wave, near field communications (NFC), ultraband, Zigbee, or infrared (IR).

The network 120 may be a wired network, a wireless network or include one or more wired and wireless networks. For example, the network 120 may be a long-range network (e.g., wide area network (WAN), the internet, or a cellular network,). Information may be sent, via the network 120 using any one of various long-range wireless communication protocols (e.g., TCP/IP, HTTP, 3G, 4G/LTE, or 5G/New Radio).

The patient biometric monitoring and processing apparatus 102 may include the patient biometric sensor 112, the processor 114, the UI sensor 116, the memory 118, and the transceiver 122. The patient biometric monitoring and processing apparatus 102 may continually or periodically monitor, store, process and communicate, via the network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

The patient biometric sensor 112 may include, for example, one or more sensors configured to sense a type of biometric patient biometrics. For example, the patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer or a microphone.

As described in more detail below, the patient biometric monitoring and processing apparatus 102 may be an ECG monitor for monitoring ECG signals of a heart (e.g., the heart 12). The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closedloop system in which the blood glucose data is sent to an insulin pump for calculated delivery of insulin without user intervention.

The transceiver 122 may include a separate transmitter and receiver. Alternatively, the transceiver 122 may include a transmitter and receiver integrated into a single device.

The processor 114 may be configured to store patient data, such as patient biometric data in the memory 118 acquired by the patient biometric sensor 112, and communicate the patient data, across the network 110, via a transmitter of the transceiver 122. Data from one or more other patient biometric monitoring and processing apparatus 102 may also be received by a receiver of the transceiver 122, as described in more detail below.

According to an embodiment, the patient biometric monitoring and processing apparatus 102 includes a UI sensor 116 which may be, for example, a piezoelectric sensor or a capacitive sensor configured to receive a user input, such as tapping or touching. For example, the UI sensor 116 may be controlled to implement a capacitive coupling in response to tapping or touching a surface of the patient biometric monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from the patient biometric monitoring and processing apparatus 102 when a gesture is detected.

The local computing device 106 of the system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via the network 120. Alternatively, the local computing device 106 may be a stationary or standalone device, such as a stationary base station including, for example, modem and/or router capability, a desktop or laptop computer using an executable program to communicate information between the patient biometric monitoring and processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, the remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

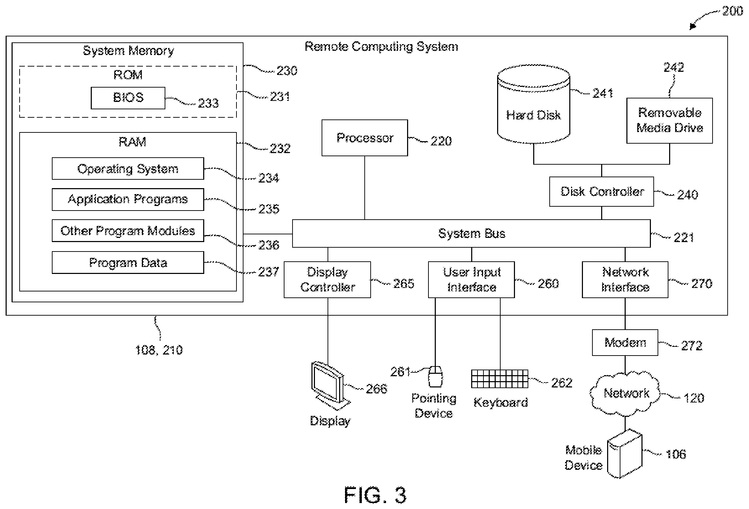

FIG. 3 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 3, computing environment 200 includes remote computing system 108 (hereinafter computer system), which is one example of a computing system upon which embodiments described herein may be implemented.

The remote computing system 108 may, via processors 220, which may include one or more processors, perform various functions. The functions may include analyzing monitored patient biometrics and the associated information and, according to physician-determined or algorithm-driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 3, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or all of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multi-processing arrangement to execute one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGS. 2 and 3, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 210 and other computers (e.g., local computing device 106).

A catheter ablation based treatment may include mapping the electrical properties of heart tissue, especially the endocardium and the heart volume, and selectively ablating cardiac tissue by application of energy. Cardiac mapping, for example creating a map of electrical potentials (a voltage map) of the wave propagation along the heart tissue or a map of arrival times (a local time activation (LAT) map) to various tissue located points, may be used for detecting local heart tissue dysfunction. Ablations, such as those based on cardiac mapping, can cease or modify the propagation of unwanted electrical signals from one portion of the heart to another.

The ablation process damages the unwanted electrical pathways by formation of non-conducting lesions. Cardiac ablation may rely on the use of three dimensional (3D) mapping systems, for example CARTO^{®}3 3D mapping system, produced by Biosense Webster, Inc. (Diamond Bar, Calif). The 3D maps can provide multiple pieces of information including visualization of tags (e.g. on a display or monitor) representing the location of electrodes of a multi-electrode catheter for an ablation session.

While the techniques described herein are not limited to any particular multi-electrode electrode catheter arrangement, the techniques may be understood in connection with the multi-electrode catheter designs 400 and 500 discussed below.

FIG. 4 shows an example showing an end effector 400 for a catheter. The end effector 400 can include a flexible circuit layer 410 extending along a longitudinal axis L- L from a proximal portion 413 to a distal portion 414, a framework 420 coupled to the flexible circuit layer 410 and extending generally parallel to the flexible circuit layer 410 along the longitudinal axis L-L from the proximal 413 to the distal portion 414, and a flexible polymer layer 430 encapsulating both the framework 420 and the flexible circuit layer 410. The flexible circuit layer 410 can include a first surface 411 and a second surface 412 opposite the first surface 411. The flexible circuit layer 410 defines a planar configuration 418 extending through the longitudinal axis L-L with the framework 420 disposed inside the flexible circuit layer 410.

In some examples, the end effector 400 can include one or more first electrodes 440a affixed to the first surface 411 of the flexible circuit layer 410, and one or more second electrodes 440b affixed to the second surface 412 of the flexible circuit layer 410. The first electrodes 440a can be axially aligned with the second electrodes 440b to define pairs of opposite facing electrodes 440. In some examples, pairs of opposite facing electrodes 440 can be aligned generally parallel to the longitudinal axis L- L. In other examples, pairs of opposite facing electrodes 440 can be aligned generally transverse to the longitudinal axis L-L. Further details of end effector 400 are set forth in U.S. Patent Application No. 18/505,956 entitled "Catheter With Flexible Polymer As Outer Support Structure," filed November 9, 2023.

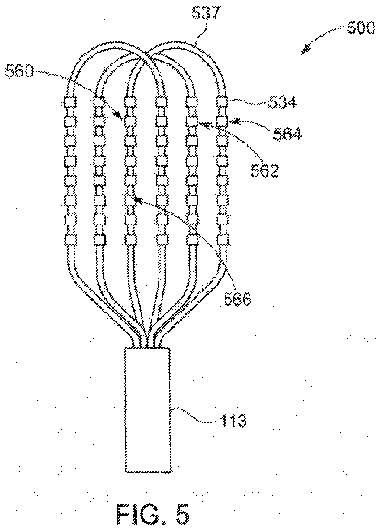

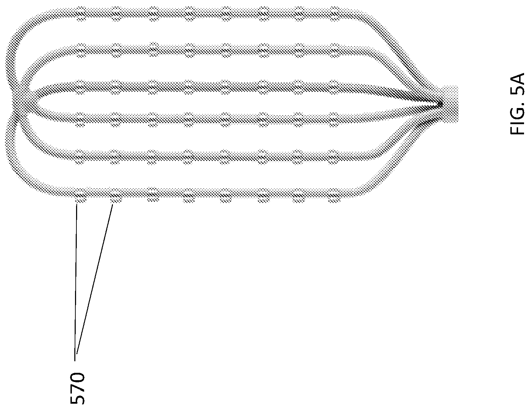

FIG. 5 shows a further example of a multi-electrode catheter design 500 for implementing techniques disclosed herein according to one or more embodiments. The catheter 500 can be a catheter having a plurality of electrodes 534, such as at least twenty (20) electrodes. As shown, the plurality of electrodes can include exactly forty eight (48) electrodes located across or dispersed on multiple spines 537. Using such a number of electrodes 534 spread across a wide area by the spines 537 allows the capturing of large amount of electrical activity over a large area at once. According to one or more embodiments, the multiple spines 537 move through a sheath in a collapsed state and may be expanded once within the patient 23 of FIG. 1 . Electrical activity at any focal point in the heart 12 may be typically measured by advancing the catheter 500, contacting the heart tissue with the catheter 500, and acquiring data related to electrical activity at that point.

As shown, catheter 500 is formed from an array of unipolar electrodes. The electrodes are considered to be unipolar, because the voltage may be measured at each electrode relative to a common electrode positioned, e.g., on the surface of the patient's body, or relative to a reference electrode on the catheter. In alternate designs, the unipolar electrodes on catheter 500 may be replaced with bi-polar electrodes 570 as illustrated in FIG. 5A. In yet other alternate designs, the unipolar electrodes on catheter 500 may be selectively paired to form bi-polar electrodes in real-time, as described in U.S. Patent Application No. 17/341,315, entitled "Bipolar Electrode Pair Selection,".

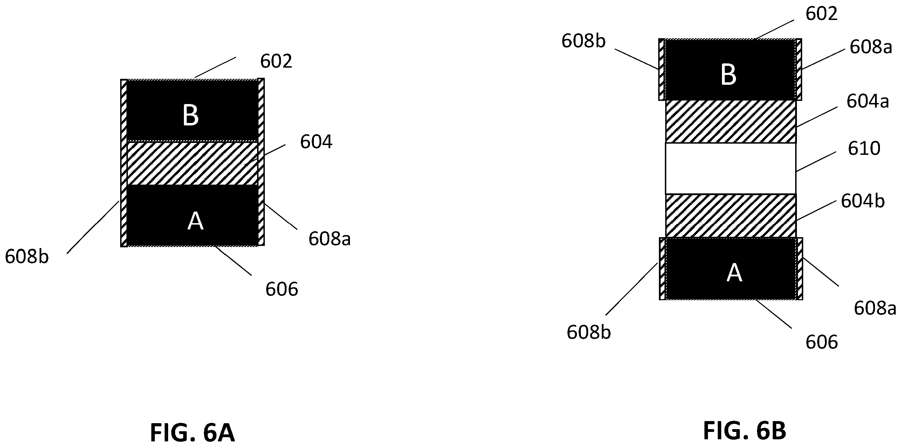

As noted above, catheter 400 includes two co-planar arrays of electrodes, one array being on each side (or face) of the catheter. In one embodiment, the electrodes on each face of catheter 400 are arranged in pairs and referred to as bipolar electrodes, because the voltage is measured between each bi-polar pair. In some arrangements, a dielectric layer 604 separates the faces of catheter 400, as shown in FIGs. 6A and 6B. In this arrangement, the bi-polar electrode pairs on each face of catheter 400 are either replaced with, or used to model, electrode pairs where the electrodes in each pair are on either side of the dielectric layer separating the faces of the catheter. An example of one such electrode pair (comprised of electrodes 602, 606) where the electrodes in the pair are on either side of the dielectric layer 604 separating the faces of the catheter is shown in FIG. 6A. A further example of one such electrode pair (comprised of electrodes 602, 606) is shown in FIG. 6B. In the example of FIG. 6B, a dielectric layer 604a, 604b is positioned adjacent to each of electrodes 602, 606, and other components 610 of the catheter (e.g., one or more catheter substrates, electrical traces, polymer or other materials) are positioned between dielectric layers 604a, 604b. In the electrode arrangements shown in FIGs. 6A and 6B, dielectric material 608a, 608b are optionally disposed along the sidewalls of electrodes 602, 606.

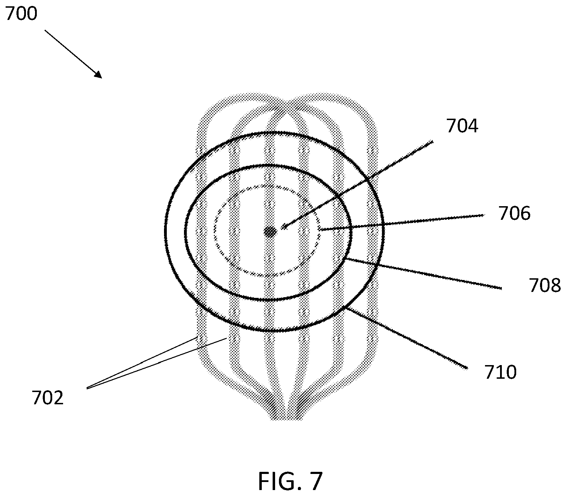

Referring now to FIG. 7, a further multi-electrode catheter 700 with a plurality of bi-polar split electrodes 702 is shown. Each bi-polar split electrode 702 is comprised of two electrodes that are spaced closely apart. While one face of multi-catheter 700 is shown as having a plurality of bi-polar split electrodes 702, in some embodiments, multi-catheter 700 has a plurality of bi-polar split electrodes 702 on both the face shown and on an opposing face (not shown). In the example of FIG. 7, bipolar split electrode 704 on catheter 700 is proximate to (or even in contact with) cardiac tissue, while the other bi-polar split electrodes on the catheter are spaced apart from bi-polar split electrode 704 that is proximate or in contact with the cardiac tissue. In the example, bi-polar split electrode 704 (the selected electrode in this example) is proximate to (or in contact with) cardiac tissue at a first location in the heart. The bi-polar split electrodes along or within the smallest ring 706 around the selected bi-polar split electrode 704 are within a first distance of the selected electrode; the bi-polar split electrodes in the middle ring 708 are within a second (further) distance of the selected electrode; and the bi-polar split electrodes in the outermost ring 710 are within a third (still further) distance of the selected electrode.

Referring still to FIG. 7, in one example, the disclosed techniques use bi-polar split electrodes positioned along or within multiple circles, or other groups of electrodes spatially positioned around the selected bi-polar split electrode 704, to identify and then subtract far field signal components from signals received by the selected electrode 704. For example, a first spatial electrode signal analysis of the electrical activity sensed by the selected electrode 704 is performed based on signals from the set of electrodes that are positioned along or within circle 706 and that are within the first distance (e.g., a radius of ring 706) of the selected electrode, a second spatial electrode signal analysis of the electrical activity sensed by the selected electrode 704 is performed based on signals from the set of electrodes that are positioned along or within circle 708 and that are within the second distance (e.g., a radius of ring 708) of the selected electrode. In some cases, a third spatial electrode signal analysis of the electrical activity sensed by the selected electrode 704 is performed based on signals from the set of electrodes that are positioned along or within circle 710 and that are within the third distance (e.g., a radius of ring 710) of the selected electrode. It is expected that the amplitude (or other characteristics) of the far field signals received by the various electrodes will vary depending on the distance of each of the various electrodes from the selected electrode. In some examples, for each distance around the selected electrode 704, the far-field component of the signal received at selected electrode 704 is determined using the techniques disclosed in U.S. Patent Publ. No. US2023/0181087 by performing a signal analysis on the signal received at selected electrode 704 and each of the signals received from electrodes positioned along or within a circle and that are within a given distance of the selected electrode 704. The disclosed techniques exploit the different characteristics of the far field signals received at multiple distances from the selected electrode to more accurately estimate, and treat the far field signals for the signals collected by the selected electrode in the desired manner, whether by scaling, subtracting, or modifying the contribution of the far field component to the electrical attributed to the selected electrode. Based on the different characteristics of the far field signals received at multiple distances from the selected electrode 704, an estimated far-field signal determined for the selected electrode 704 is subtracted from the voltage detected by selected electrode 704 to estimate the near-field signal received by the selected electrode 704. The above techniques will be referred to later in this disclosure as "multi-polar" techniques. These techniques can be repeated for all bi-polar split electrodes of the catheter until all of the electrodes have been processed.

The above techniques may be applied at different locations within the heart as the catheter moves through a patient's heart during a procedure in order to generate improved electro-anatomical maps of the heart. In one example, first and second electro-anatomical maps of the heart are generated, where the first electroanatomical map depicts electrical activity identified based on the first spatial electrode analysis and the second electro-anatomical map depicts electrical activity identified based on the second spatial electrode signal analysis. Alternatively, the first electroanatomical map depicts scar tissue (e.g., areas of low conductivity) identified based on the first spatial electrode analysis and the second electro-anatomical map depicts scar tissue (e.g., areas of low conductivity) identified based on the second spatial electrode signal analysis.

While in some examples, the above techniques collect signals by placing the multi-catheter electrode in the heart to collect signals, in other examples the catheter is placed on the epicard or on the heart but outside of it.

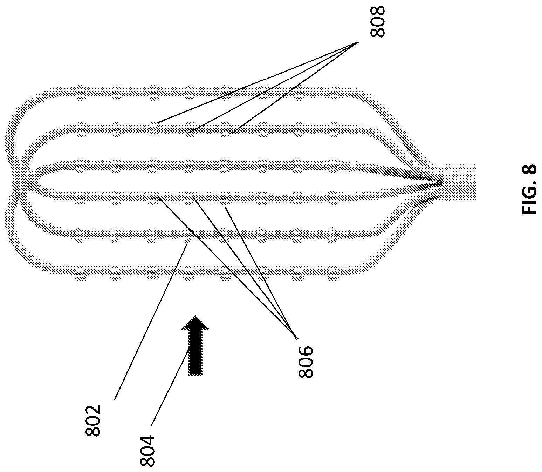

While the example described above was based on the use of bi-polar split electrodes spaced within multiple concentric rings around the selected electrode, the techniques disclosed herein may applied using other individual or groups of electrodes spaced different distances from the selected electrode. For example, consider a case shown in FIG. 8 where the selected bi-polar split electrode 802 is proximate to (or in contact with) cardiac tissue at a first location in the heart, and signals emanate from the first location in a direction of propagation illustrated by arrow 804. In such a case, a first set of bi-polar electrodes 806 are spaced at or within a first distance from the selected electrode 802, and a second set of bi-polar split electrodes 808 are spaced at or within a second distance from the selected electrode. As shown, the first set of electrodes 806 are aligned at, or with respect to, an angle perpendicular to the direction of propagation illustrated by arrow 804. Similarly, the second set of electrodes 808 are aligned at, or with respect to, an angle perpendicular to the direction of propagation illustrated by arrow 804.

In examples where the techniques are applied using catheter 400 (which has two co-planar arrays of bi-polar electrodes, one array being on each side (or face) of the catheter), the first set of electrodes and the second set of electrodes that are spaced, respectively, the first and second distance from the selected electrode, are arranged on separate planes and separated, for example, by a dielectric layer. In some examples, these techniques are applied using a multi-electrode catheter that comprises bi-polar electrodes which are spaced apart from each other by a distance which is smaller than dimensions of each electrode in the pair.

The above techniques are particularly applicable to unipolar and bi-polar signals which are a combination of near and far field signals. During a cardiac ablation procedure, it may be desirable to isolate the near field and far signals. The above techniques may be applied to decompose signals into near field and far field to allow identification of only near field activity, identification of only far field activity, or identification of varying proportions of each. In some examples, a machine learning algorithm is trained and applied to decompose near field and far field signals using the above-described techniques.

The signal diagram set forth below depicts an example of application of the above techniques for six unipolar electrodes (identified as B1 to B6) on a multi-electrode catheter such as catheter 500. The diagram depicts six pairs 902, 904, 906, 908, 910, 912 of signals, where the boxed area for each signal pair represents a region of interest. Each pair of signals 902, 904, 906, 908, 910, 912 includes a gray signal that corresponds to the signal collected from a particular selected electrode (e.g., B1), and a white signal that represents signals collected from one or more electrodes (or a set of electrodes) that are spaced along or within a given distance from such electrode (e.g, B1). According to the techniques described herein, there may be other signals (not shown) for the particular electrode (e.g., B1) corresponding to signals collected from one or more other electrodes (or other set of electrodes) that are spaced along or within a different distance from such electrode (e.g, B1).

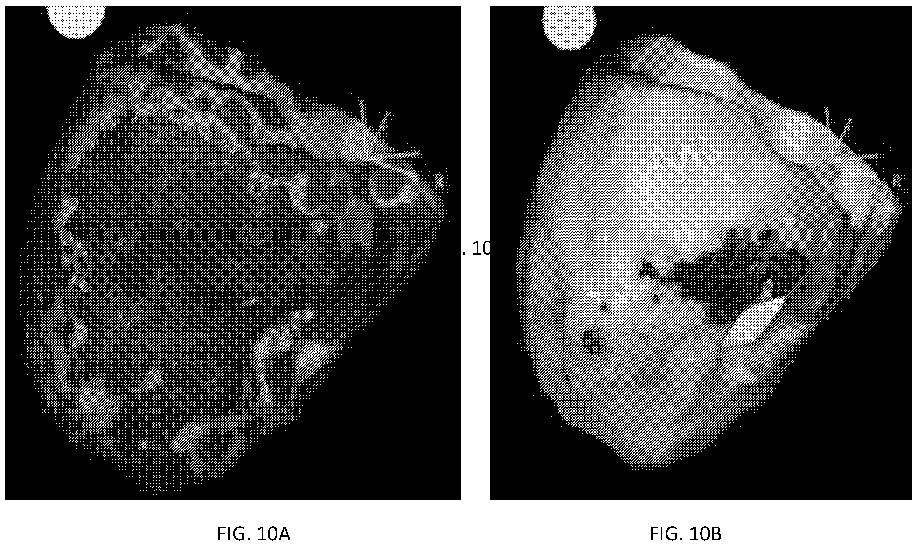

As described above, the disclosed techniques may be applied to decompose the signal collected at each electrode (e.g., B1-B6) into near field and far field components for the purpose identifying only near field activity at each such electrode, identifying only far field activity at each such electrode, or identifying varying proportions of each at each electrode. FIGs. 10A and 10B are two electro-anatomical maps generated using such techniques, where each map reflects a different proportion of near field and far field signals captured by each electrode (e.g., B1-B6).

In certain embodiments, the electro-anatomical mapping system generates at least maps depicting electrical activity, with each map reflecting a variable degree of far-field signal contribution. For example, the system may provide: (1) a near-field map, where the electrograms that define points in the map reflect primarily local electrical activity with minimal far-field signal interference (e.g., by scaling down or subtracting the far field components), (2) a near-far-field map, where the electrograms that define points in the maps have far-field components have been scaled or subtracted moderately, and (3) a far-field map, where the electrograms include more relatively more far-field signal components; or a continuum of maps and/or optional mapping modes in which varying degrees of far-field and near-field signals define the points therein. This variability in signal processing and map creation processing may provide physicians with critical insights into the underlying arrhythmia, potentially revealing aspects of the electrical source or propagation that may not be discernible from near-field activity alone. By visualizing electrical behavior across different levels of far-field contribution, physicians may be able to better differentiate between arrhythmic sources, thereby improving diagnostic accuracy and treatment outcomes. Additional maps beyond three may be provided as desired to allow even more granular variability in the far-field signal contribution, as permitted by the number and arrangement of electrodes.

The electro-anatomical mapping system may offer intuitive methods for selecting among the various map options through its user interface (UI) and user experience (UX) design. One possible approach is to allow the operator to choose from a predefined menu, presenting options such as "near-field," "near-far-field," and "far-field" maps. Another approach could leverage a dynamic scrolling feature, similar to zooming in or out with a mouse or trackpad or other gestures. By scrolling, the user could transition between maps, progressively adjusting the contribution of far-field signals. For instance, scrolling upward might reveal maps with progressively less far-field interference, moving toward near-field activity, while scrolling downward might increase the far-field contribution. These selectable options provide flexibility and ease of use, enabling the operator to quickly navigate or "animate" between maps to allow improved diagnostics.

The disclosed techniques may be used to generate real time maps using signals collected in a single snapshot in time. Alternatively, signals may be collected and processed over a longer time window before the data is displayed.

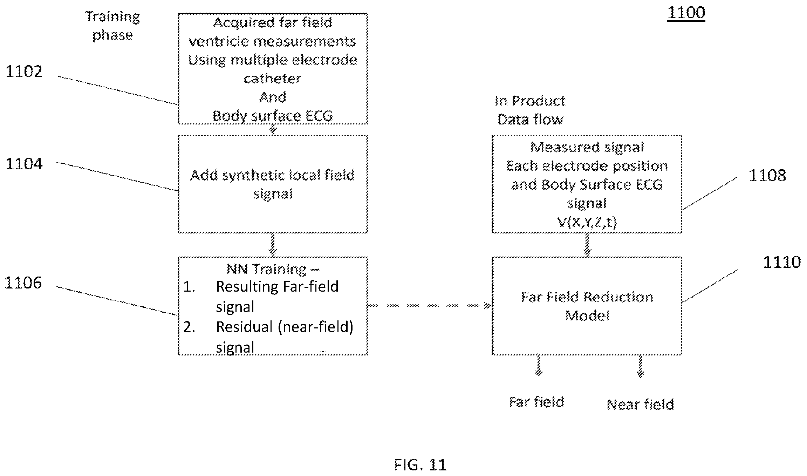

In some embodiments, a machine learning algorithm is trained to decompose near field and far field signals using the above-described techniques. FIG. 11 illustrates an exemplary method 1100 for training a machine learning algorithm (steps 1102-1106) and applying the machine learning algorithm (step 1110) to decompose near and far field components from signals measured in step 1108. In one example of step 1102, training data comprising far field ventricular measurements are acquired using a multi-catheter electrode and a body surface ECG. In some examples, the training data set may include isolated far field signals obtained by, e.g., placing the catheter in a position where it is not in contact with the heart muscle or within a proximity thereto as identified by, e.g., a tissue proximity index (TPI) value. The signals for the training data set may also include ECG values for a given 3D location (per each electrode). Surface ECG signals can also be used for part of the training signals. Surface ECG signals are considered basically as far field signals. In step 1104, training data comprising synthetic local field signals is added to the training data acquired in step 1102. For example, previously identified near-field signals can be modified by varying the amplitude and/or adding random noise in order to generate synthetic local field signals for use in step 1104. In some examples, ground-truth data for far-field signals is obtained by performing multiple cryothermal peels characterized by freezing of the myocardial layers from deep up towards the mapping surface (such that, e.g., far-field contributions in the frozen layers are silenced). This ground truth data can be used for training the machine learning algorithm, including training the neural network to identify near-field and far-field components of signals generated at different depths from the tissue surface. The present disclosure encompasses any suitable method or source for collecting or creating relevant data for training the machine learning algorithm. Neural network training is performed in step 1106 using a resulting far-field signal captured using the body surface ECG and the residual near-field signal that remains after the far-field signal is subtracted from a signal captured by the multi-electrode catheter. The training results in a far field reduction model 1110 that accepts as it inputs patient measurements acquired using a multi-catheter electrode and a body surface ECG (step 1108) and decomposes the measured signals from the catheter electrodes into far field and near field components.

Unlike generic data processing, the disclosed neural network is trained using electrophysiology-specific data, including synthetic and/or clinical signals, thereby improving computer functionality in this technical field. The result is enhanced diagnostic accuracy and improved real-time mapping performance that cannot be achieved by human review or by general-purpose computer routines.

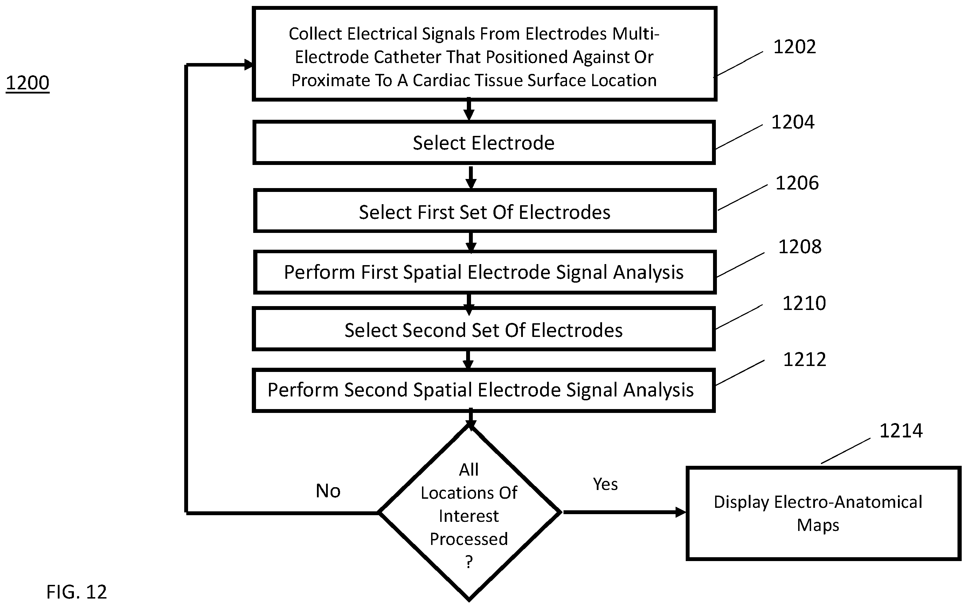

FIG. 12 is a flow diagram illustrating a method 1200 for generating electroanatomical maps of the heart depicting electrical activity of the cardiac tissue or scar tissue based on the spatial electrode analysis techniques disclosed herein according to one or more embodiments. In step 1202, electrical activity is received from a plurality of electrodes of a multi-electrode catheter within a heart of a patient. In step 1204, an electrode in contact with or proximate to first location of cardiac tissue is selected from the plurality of electrodes. In step 1206, a first set of electrodes is selected, each of which is at or within a first distance of the first electrode, wherein the first set of electrodes does not include the first electrode. In step 1208, a first spatial electrode signal analysis is performed of the electrical activity of the first electrode based on signals from the first set of electrodes. In step 1210, a second set of electrodes is selected each of which is within a second distance of the first electrode, the second distance being greater than the first distance, wherein the second set of electrodes does not include the first electrode. In step 1212, a second spatial electrode signal analysis is performed of the electrical activity of the first electrode in accordance with signals from the second set of electrodes. This process is repeated for a plurality of further locations within the heart until all locations of interest have been processed. In step 1214, the results of the first and second spatial analysis at each of the first location and each of the further locations are used to generate at least first and second electro-anatomical maps of the heart. The maps depict electrical activity of the cardiac tissue corresponding to the first location and the plurality of further locations, wherein the first electro-anatomical map depicts electrical activity identified based on the first spatial electrode analysis and the second electroanatomical map depicts electrical activity identified based on the second spatial electrode signal analysis. Alternatively, the first electro-anatomical map depicts scar tissue identified based on the first spatial electrode analysis and the second electroanatomical map depicts scar tissue identified based on the second spatial electrode signal analysis.

Although features and elements are described above in particular combinations, one of ordinary skills in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A system (10) comprising:
one or more processors in communication with a plurality of electrodes (26) of a multi-electrode catheter (14);
a display (27) ; and
a memory in communication with the display and the one or more processors;
wherein the one or more processors are collectively configured to perform the following for a plurality of locations of cardiac tissue in a heart (12):
receive (1202) electrical activity of a heart of a patient from a plurality of electrodes of a multi-electrode catheter;
identify a selected electrode by selecting (1204) an electrode from the plurality of electrodes;
select (1206) a first set of electrodes (806) each of which is within a first distance of the selected electrode, wherein the first set of electrodes does not include the selected electrode;
perform (1208) a first spatial electrode signal analysis of the electrical activity of the selected electrode in accordance with signals from the first set of electrodes;
select (1210) a second set of electrodes (808) each of which is within a second distance of the selected electrode, the second distance being greater than the first distance, wherein the second set of electrodes does not include the selected electrode; and
performing (1212) a second spatial electrode signal analysis of the electrical
activity of the selected electrode in accordance with signals from the second set of electrodes; and
wherein the one or more processors are further collectively configured to generate at least a first electro-anatomical map of the heart depicting electrical activity of cardiac tissue corresponding to each of the plurality of locations, and a second electro-anatomical map of the heart depicting electrical activity of cardiac tissue corresponding to each of the plurality of further locations, wherein the first electro-anatomical map depicts electrical activity or scar tissue identified based on the first spatial electrode signal analysis and the second electro-anatomical map depicts electrical activity or scar tissue identified based on the second spatial electrode signal analysis.

2. The system of claim 1, wherein the first spatial electrode signal analysis and the second spatial signal analysis are performed using a previously trained neural network.

3. The system of claim 1, wherein the first set of electrodes and the second set of electrodes are separated by a dielectric layer (604).

4. The system of claim 1, wherein the first set of electrodes and the second set of electrodes are arranged on different planes.

5. The system of claim 1, wherein the first spatial electrode signal analysis is based at least in part on the first distance, and the second spatial electrode signal analysis is based at least part on the second distance.

6. The system of claim 5, wherein far field signals identified based on the first spatial analysis and the second spatial analysis, wherein said far field signals are subtracted from electrical signals from the first electrode, and wherein said far field signals are determined from the signals from the first set (806) of electrodes based on the first distance and from the signals from the second set of electrodes (808) based on second first distance.

7. The system of claim 1, wherein the first electrode is at a first location within the heart.

8. The system of claim 7, where signals emanate from the first location along a first direction, and the first set of electrodes (806) and the second set of electrodes (808) are aligned at an angle perpendicular to said first direction.

9. The system of claim 1, wherein the multi-electrode catheter (14) comprises at least one pair of electrodes (26) which are spaced apart from each other by a distance which is smaller than dimensions of the electrodes in the pair.

10. A computer-readable storage medium comprising program code for causing a computer to perform the following for a plurality of locations of cardiac tissue in a heart (12):
Receive (1202) electrical activity of a heart of a patient from a plurality of electrodes (26) of a multi-electrode catheter (14);
identify a selected electrode by selecting (1204) an electrode from the plurality of electrodes;
select (1206) a first set of electrodes (806) each of which is within a first distance of the selected electrode, wherein the first set of electrodes does not include the selected electrode;
perform (1208) a first spatial electrode signal analysis of the electrical activity of the selected electrode in accordance with signals from the first set of electrodes;
select (1210) a second set of electrodes (808) each of which is within a second distance of the selected electrode, the second distance being greater than the first distance, wherein the second set of electrodes does not include the selected electrode; and
performing (1212) a second spatial electrode signal analysis of the electrical activity of the selected electrode in accordance with signals from the second set of electrodes; and
wherein the computer-readable storage medium further comprises program code for causing a computer to generate at least first and second electro-anatomical maps of the heart depicting electrical activity of the cardiac tissue corresponding to the plurality of locations, wherein the first electro-anatomical map depicts electrical activity or scar tissue identified based on the first spatial electrode analysis and the second electro-anatomical map depicts electrical activity or scar tissue identified based on the second spatial electrode signal analysis.

## Patentansprüche

1. System (10), umfassend:
einen oder mehrere Prozessoren, die mit einer Vielzahl von Elektroden (26) eines Mehrfachelektrodenkatheters (14) in Verbindung stehen;
eine Anzeige (27); und
einen Speicher, der mit der Anzeige und dem einen oder den mehreren Prozessoren in Verbindung steht; wobei der eine oder die mehreren Prozessoren gemeinsam konfiguriert sind, um für eine Vielzahl von Stellen von Herzgewebe in einem Herzen (12) Folgendes durchzuführen:
Empfangen (1202) der elektrischen Aktivität eines Herzens eines Patienten von einer Vielzahl von Elektroden eines Mehrfachelektrodenkatheters;
Identifizieren einer ausgewählten Elektrode durch Auswählen (1204) einer Elektrode aus der Vielzahl von Elektroden;
Auswählen (1206) eines ersten Satzes von Elektroden (806), von denen sich jede in einem ersten Abstand zur ausgewählten Elektrode befindet, wobei der erste Satz von Elektroden die ausgewählte Elektrode nicht einschließt;
Durchführen (1208) einer ersten räumlichen Elektrodensignalanalyse der elektrischen Aktivität der ausgewählten Elektrode gemäß Signalen von dem ersten Satz von Elektroden;
Auswählen (1210) eines zweiten Satzes von Elektroden (808), von denen sich jede innerhalb eines zweiten Abstands von der ausgewählten Elektrode befindet, wobei der zweite Abstand größer als der erste Abstand ist, wobei der zweite Satz von Elektroden die ausgewählte Elektrode nicht einschließt; und
Durchführen (1212) einer zweiten räumlichen Elektrodensignalanalyse der elektrischen Aktivität der ausgewählten Elektrode gemäß Signalen aus dem zweiten Satz von Elektroden; und
wobei der eine oder die mehreren Prozessoren ferner gemeinsam konfiguriert sind, um mindestens eine erste elektroanatomische Karte des Herzens, die elektrische Aktivität von Herzgewebe entsprechend jedem der Vielzahl von Orten darstellt, und eine zweite elektroanatomische Karte des Herzens, die elektrische Aktivität von Herzgewebe entsprechend jedem der Vielzahl von weiteren Orten darstellt, zu erzeugen, wobei die erste elektroanatomische Karte elektrische Aktivität oder Narbengewebe darstellt, die bzw. das basierend auf der ersten räumlichen Elektrodensignalanalyse identifiziert wurde, und die zweite elektroanatomische Karte elektrische Aktivität oder Narbengewebe darstellt, die bzw. das basierend auf der zweiten räumlichen Elektrodensignalanalyse identifiziert wurde.

2. System nach Anspruch 1, wobei die erste räumliche Elektrodensignalanalyse und die zweite räumliche Signalanalyse unter Verwendung eines zuvor trainierten neuronalen Netzwerks durchgeführt werden.

3. System nach Anspruch 1, wobei der erste Satz von Elektroden und der zweite Satz von Elektroden durch eine dielektrische Schicht (604) getrennt sind.

4. System nach Anspruch 1, wobei der erste Satz von Elektroden und der zweite Satz von Elektroden auf verschiedenen Ebenen angeordnet sind.

5. System nach Anspruch 1, wobei die erste räumliche Elektrodensignalanalyse zumindest teilweise auf dem ersten Abstand basiert und die zweite räumliche Elektrodensignalanalyse zumindest teilweise auf dem zweiten Abstand basiert.

6. System nach Anspruch 5, wobei Fernfeldsignale basierend auf der ersten räumlichen Analyse und der zweiten räumlichen Analyse identifiziert werden, wobei die Fernfeldsignale von elektrischen Signalen von der ersten Elektrode subtrahiert werden, und wobei die Fernfeldsignale aus den Signalen von dem ersten Satz (806) von Elektroden basierend auf dem ersten Abstand und aus den Signalen von dem zweiten Satz von Elektroden (808) basierend auf dem zweiten ersten Abstand bestimmt werden.

7. System nach Anspruch 1, wobei sich die erste Elektrode an einer ersten Stelle innerhalb des Herzens befindet.

8. System nach Anspruch 7, wobei Signale von dem ersten Ort entlang einer ersten Richtung ausgehen und der erste Satz von Elektroden (806) und der zweite Satz von Elektroden (808) in einem Winkel senkrecht zu der ersten Richtung ausgerichtet sind.

9. System nach Anspruch 1, wobei der Mehrfachelektrodenkatheter (14) mindestens ein Paar von Elektroden (26) umfasst, die um einen Abstand voneinander beabstandet sind, der kleiner als Abmessungen der Elektroden in dem Paar ist.

10. Computerlesbares Speichermedium, umfassend Programmcode, um einen Computer zu veranlassen, für eine Vielzahl von Stellen von Herzgewebe in einem Herz (12) Folgendes durchzuführen:
Empfangen (1202) elektrischer Aktivität eines Herzens eines Patienten von einer Vielzahl von Elektroden (26) eines Mehrfachelektrodenkatheters (14);
Identifizieren einer ausgewählten Elektrode durch Auswählen (1204) einer Elektrode aus der Vielzahl von Elektroden;
Auswählen (1206) eines ersten Satzes von Elektroden (806), von denen sich jede in einem ersten Abstand zur ausgewählten Elektrode befindet, wobei der erste Satz von Elektroden die ausgewählte Elektrode nicht einschließt;
Durchführen (1208) einer ersten räumlichen Elektrodensignalanalyse der elektrischen Aktivität der ausgewählten Elektrode gemäß Signalen von dem ersten Satz von Elektroden;
Auswählen (1210) eines zweiten Satzes von Elektroden (808), von denen sich jede innerhalb eines zweiten Abstands von der ausgewählten Elektrode befindet, wobei der zweite Abstand größer als der erste Abstand ist, wobei der zweite Satz von Elektroden die ausgewählte Elektrode nicht einschließt; und
Durchführen (1212) einer zweiten räumlichen Elektrodensignalanalyse der elektrischen Aktivität der ausgewählten Elektrode gemäß Signalen aus dem zweiten Satz von Elektroden; und
wobei das computerlesbare Speichermedium ferner Programmcode umfasst, um einen Computer zu veranlassen, mindestens erste und zweite elektroanatomische Karten des Herzens zu erzeugen, die elektrische Aktivität des Herzgewebes darstellen, die der Vielzahl von Orten entspricht, wobei die erste elektroanatomische Karte elektrische Aktivität oder Narbengewebe darstellt, die bzw. das basierend auf der ersten räumlichen Elektrodenanalyse identifiziert wurde, und die zweite elektroanatomische Karte elektrische Aktivität oder Narbengewebe darstellt, die bzw. das basierend auf der zweiten räumlichen Elektrodensignalanalyse identifiziert wurde.

## Revendications

1. Système (10) comprenant :
un ou plusieurs processeurs en communication avec une pluralité d'électrodes (26) d'un cathéter multi-électrodes (14) ;
un dispositif d'affichage (27) ; et
une mémoire en communication avec le dispositif d'affichage et le ou les processeurs ; dans lequel le ou les processeurs sont collectivement configurés pour effectuer ce qui suit pour une pluralité d'emplacements de tissu cardiaque dans un cœur (12) :
la réception (1202) d'une activité électrique d'un cœur d'un patient à partir d'une pluralité d'électrodes d'un cathéter multi-électrodes ;
l'identification d'une électrode sélectionnée en sélectionnant (1204) une électrode parmi la pluralité d'électrodes ;
la sélection (1206) d'un premier ensemble d'électrodes (806) dont chacune est située à une première distance de l'électrode sélectionnée, dans lequel le premier ensemble d'électrodes ne comporte pas l'électrode sélectionnée ;
la réalisation (1208) d'une première analyse spatiale de signal d'électrode de l'activité électrique de l'électrode sélectionnée conformément à des signaux provenant du premier ensemble d'électrodes ;
la sélection (1210) d'un second ensemble d'électrodes (808) dont chacune est située à une seconde distance de l'électrode sélectionnée, la seconde distance étant supérieure à la première distance, dans lequel le second ensemble d'électrodes ne comporte pas l'électrode sélectionnée ; et
la réalisation (1212) d'une seconde analyse spatiale de signal d'électrode de l'activité électrique de l'électrode sélectionnée conformément à des signaux provenant du second ensemble d'électrodes ; et
dans lequel le ou les processeurs sont en outre collectivement configurés pour générer au moins une première carte électro-anatomique du cœur représentant une activité électrique de tissu cardiaque correspondant à chacun de la pluralité d'emplacements, et une seconde carte électro-anatomique du cœur représentant une activité électrique de tissu cardiaque correspondant à chacun de la pluralité d'autres emplacements, dans lequel la première carte électro-anatomique représente une activité électrique ou un tissu cicatriciel identifié sur la base de la première analyse spatiale du signal d'électrode et la seconde carte électro-anatomique représente une activité électrique ou un tissu cicatriciel identifié sur la base de la seconde analyse spatiale du signal d'électrode.

2. Système selon la revendication 1, dans lequel la première analyse spatiale de signal d'électrode et la seconde analyse spatiale de signal sont effectuées à l'aide d'un réseau neuronal préalablement entraîné.

3. Système selon la revendication 1, dans lequel le premier ensemble d'électrodes et le second ensemble d'électrodes sont séparés par une couche diélectrique (604).

4. Système selon la revendication 1, dans lequel le premier ensemble d'électrodes et le second ensemble d'électrodes sont disposés sur des plans différents.

5. Système selon la revendication 1, dans lequel la première analyse spatiale de signal d'électrode est basée au moins en partie sur la première distance, et la seconde analyse spatiale de signal d'électrode est basée au moins en partie sur la seconde distance.

6. Système selon la revendication 5, dans lequel les signaux de champ lointain sont identifiés sur la base de la première analyse spatiale et de la seconde analyse spatiale, dans lequel lesdits signaux de champ lointain sont soustraits des signaux électriques de la première électrode, et dans lequel lesdits signaux de champ lointain sont déterminés à partir des signaux provenant du premier ensemble (806) d'électrodes sur la base de la première distance et à partir des signaux provenant du second ensemble d'électrodes (808) sur la base de la seconde première distance.

7. Système selon la revendication 1, dans lequel la première électrode est située au niveau d'un premier emplacement à l'intérieur du cœur.

8. Système selon la revendication 7, où des signaux émanent du premier emplacement le long d'une première direction, et le premier ensemble d'électrodes (806) et le second ensemble d'électrodes (808) sont alignés selon un angle perpendiculaire à ladite première direction.

9. Système selon la revendication 1, dans lequel le cathéter multi-électrodes (14) comprend au moins une paire d'électrodes (26) qui sont espacées l'une de l'autre d'une distance qui est inférieure aux dimensions des électrodes dans la paire.

10. Support de stockage lisible par ordinateur comprenant un code de programme pour amener un ordinateur à effectuer ce qui suit pour une pluralité d'emplacements de tissu cardiaque dans un cœur (12) :
la réception(1202) d'une activité électrique d'un cœur d'un patient à partir d'une pluralité d'électrodes (26) d'un cathéter multi-électrodes (14) ;
l'identification d'une électrode sélectionnée en sélectionnant (1204) une électrode parmi la pluralité d'électrodes ;
la sélection (1206) d'un premier ensemble d'électrodes (806) dont chacune est située à une première distance de l'électrode sélectionnée, dans lequel le premier ensemble d'électrodes ne comporte pas l'électrode sélectionnée ;
la réalisation (1208) d'une première analyse spatiale de signal d'électrode de l'activité électrique de l'électrode sélectionnée conformément à des signaux provenant du premier ensemble d'électrodes ;
la sélection (1210) d'un second ensemble d'électrodes (808) dont chacune est située à une seconde distance de l'électrode sélectionnée, la seconde distance étant supérieure à la première distance, dans lequel le second ensemble d'électrodes ne comporte pas l'électrode sélectionnée ; et
la réalisation (1212) d'une seconde analyse spatiale de signal d'électrode de l'activité électrique de l'électrode sélectionnée conformément à des signaux provenant du second ensemble d'électrodes ; et
dans lequel le support de stockage lisible par ordinateur comprend en outre un code de programme pour amener un ordinateur à générer au moins des première et seconde cartes électro-anatomiques du cœur représentant une activité électrique de tissu cardiaque correspondant à la pluralité d'emplacements, dans lequel la première carte électro-anatomique représente une activité électrique ou un tissu cicatriciel identifié sur la base de la première analyse spatiale d'électrode et la seconde carte électro-anatomique représente une activité électrique ou un tissu cicatriciel identifié sur la base de la seconde analyse spatiale de signal d'électrode.
